(19) Europäisches Patentamt
European Patent Office
Office européen des brevets

(11) Veröffentlichungsnummer: **0 124 486**
**B1**

(12) # EUROPÄISCHE PATENTSCHRIFT

(45) Veröffentlichungstag der Patentschrift: **25.02.87**

(51) Int. Cl.⁴: **C 08 K 5/34**, C 07 D 211/46

(21) Anmeldenummer: **84810201.8**

(22) Anmeldetag: **27.04.84**

(54) **Verwendung von Dipiperidin-di-carbamaten zum Stabilisieren von synthetischen Polymeren.**

(30) Priorität: **03.05.83 IT 2090083**

(43) Veröffentlichungstag der Anmeldung: **07.11.84 Patentblatt 84/45**

(45) Bekanntmachung des Hinweises auf die Patenterteilung: **25.02.87 Patentblatt 87/9**

(84) Benannte Vertragsstaaten: **BE CH DE FR GB IT LI NL**

(56) Entgegenhaltungen: **EP-A-0 031 304**

(73) Patentinhaber: **CIBA- GEIGY S.p.A., Strada Statale 233- Km. 20,5, Origgio (IT)**

(72) Erfinder: **Cantatore, Giuseppe, Dr., Via Generale Planelli, 68, I-70032 Bitonto (Bari) (IT)**

(74) Vertreter: **Stamm, Otto, Patentabteilung der CIBA-GEIGY AG Postfach, CH- 4002 Basel (CH)**

**EP 0 124 486 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft die Verwendung bestimmter Dipiperidin-di-carbamate zum Stabilisieren von synthetischen Polymeren.

Dipiperidin-di-carbamate der Formel

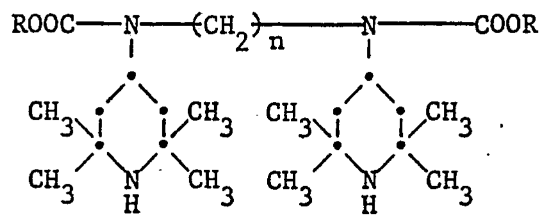

worin R Ethyl und n 3 oder 6 bedeuten sind aus EP-A-31304 als Zwischenprodukte für die Herstellung von als Lichtschutzmittel für Polymere zu verwendenden Verbindungen derselben allgemeinen Formel, worin R ein Polyalkylpiperidylrest ist, bekannt. Es ist nun gefunden worden, dass diese Verbindungen überraschenderweise selbst stabilisierende Wirkung in Polymeren zeigen, sogar mit besseren Effekten als die in der oben erwähnten Publikation beschriebenen Endprodukte.

Gegenstand der vorliegenden Erfindung ist demnach die Verwendung von Dipiperidinverbindungen der Formel I

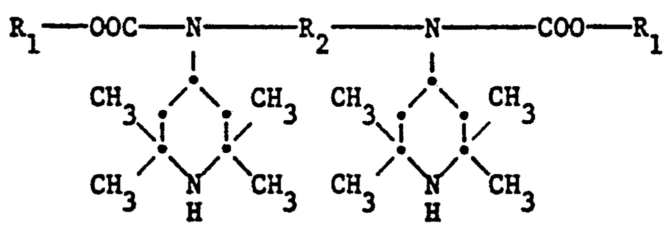

(I)

worin $R_1$ $C_1$-$C_{12}$ Alkyl, $C_3$-$C_{12}$ Alkenyl, $C_5$-$C_{12}$ Cylcoalkyl, unsubstituiertes oder durch 1 bis 3 $C_1$-$C_4$ Alkylgruppen substituiertes Phenyl oder Hydroxyphenyl, unsubstituiertes oder durch 1 bis 3 $C_1$-$C_4$ Alkylgruppen substituiertes Benzyl oder Hydroxybenzyl ist und $R_2$ unsubstituiertes oder durch -OH substituiertes $C_1$-$C_{18}$ Alkylen, $C_5$-$C_{18}$ Cycloalkylen, $C_6$-$C_{18}$ Arylen, $C_7$-$C_{18}$ Aralkylen oder eine Gruppe der Formel

$$-R_3-(X-R_4)_n- \qquad (II)$$

bedeutet, worin $R_3$ und $R_4$ unabhängig voneinander $C_2$-$C_6$ Alkylen sind, n 1 oder 2 bedeutet und X -O- oder -N($R_5$)- ist, wobei $R_5$ $C_1$-$C_{12}$ Alkyl, $C_5$-$C_{12}$ Cycloalkyl, $C_6$-$C_{12}$ Aryl, $C_7$-$C_{12}$ Aralkyl, 2,2,6,6-Tetramethyl-piperidin-4-yl oder eine Gruppe -$COOR_1$ bedeutet, worin $R_1$ die oben angegebene Bedeutung hat, zum Stabilisieren von synthetischen Polymeren gegen die Einwirkung von Licht, Wärme und Sauerstoff.

Die Bedeutung der verschiedenen Reste speziell erläuternde Beispiele sind wie folgt:

Für $R_1$: Methyl, Ethyl, Propyl, Isopropyl, Butyl, tert.-Butyl, sek.-Butyl, Hexyl, 2-Ethylhexyl, Octyl, 1,1,3,3-Tetramethylbutyl, Decyl, Dodecyl, Allyl, Methallyl, But-2-enyl, Undec-10-enyl, Cyclohexyl, 2- oder 4-Methylcyclohexyl, 3,3,5-Trimethylcyclohexyl, Cyclooctyl, Cyclododecyl, Phenyl, o-, m- und p-Methylphenyl, 2,6-Dimethylphenyl, 2,4,6-Trimethylphenyl, 4-t-Butylphenyl, 4-t-Octylphenyl, 4-Methoxyphenyl, 4-Ethoxyphenyl, 3,5-Di-t-butyl-4-hydroxyphenyl, Benzyl, 4-Methylbenzyl, 4-Hydroxybenzyl und 3,5-Di-t-butyl-4-hydroxy-benzyl;

Für $R_2$: Methylen, Ethylen, 1,2- und 1,3-Propylen, 2-Hydroxy-1,3-propylen, Tetramethylen, Pentamethylen, 2,2-Dimethyl-1,3-propylen, Hexamethylen, Decamethylen, Dodecamethylen, Cyclohexylen, Cyclohexylendimethylen, Phenylen und Xylylen;

Für $R_3$ und $R_4$: Ethylen. 1,2- oder 1,3-Propylen, Tetramethylen und Hexamethylen: und

Für $R_5$: Methyl, Ethyl, Propyl, Isopropyl, Butyl, tert.-Butyl, sek.-Butyl, Hexyl, 2-Ethylhexyl, Octyl, 1,1,3,3-Tetramethylbutyl, Decyl, Dodecyl, Cyclohexyl, 2- or 4-Methylcyclohexyl, 3,3,5-Trimethyl-cyclohexyl, Cyclooctyl, Cyclododecyl, Phenyl, 0-, m- und p-Methylphenyl, 2,6-Dimethylphenyl, 2,4,6-Trimethylphenyl, 4-t-Butylphenyl, 4-(1,1,3,3-Tetramethylbutyl)-phenyl, 4-Methoxyphenyl, 4-Ethoxyphenyl, 3,5-Di-t-butyl-4-hydroxyphenyl, Benzyl, 4-Methylbenzyl, 4-Hydroxy-benzyl, 3,5-Di-t-butyl-4-hydroxybenzyl und 2,2,6,6-Tetramethyl-piperidin-4-yl.

Diejenigen Verbindungen der Formel 1 sind bevorzugt, worin $R_1$ $C_1$-$C_8$-Alkyl,$C_3$-$C_8$ Alkenyl, Cyclohexyl, Phenyl oder Benzyl ist, $R_2C_1$-$C_6$ Alkylen, Cyclohexylen, Phenylen, Xylylen oder eine Gruppe der Formel II ist, worin $R_3$ und $R_4$ $C_2$-$C_4$ Alkylen sind und $R_5$ eine Gruppe -$COOR_1$ bedeutet, worin $R_1$ die in dieser Bevorzugung bereits angegebene Bedeutung hat.

Besonders bevorzugt sind die Verbindungen der Formel I, worin $R_1$ $C_1$-$C_4$ Alkyl und $R_2$ $C_2$-$C_6$ Alkylen bedeuten.

Die Verbindungen der Formel I, worin $R_1$ weder Methyl noch Ethyl bedeutet, d.h. worin $R_1$ $C_3$-$C_{12}$ Alkyl, $C_3$-$C_{12}$ Alkenyl, $C_5$-$C_{12}$ Cycloalkyl, unsubstituiertes oder durch 1 bis 3 $C_1$-$C_4$ Alkylgruppen substituiertes Phenyl oder Hydroxyphenyl oder unsubstituiertes oder durch 1 bis 3 $C_1$-$C_4$ Alkylgruppen substituiertes Benzyl oder Hydroxybenzyl ist sind neu und stellen demnach einen weiteren Gegenstand der vorliegenden Erfindung dar.

Die Verbindungen der Formel I können durch an sich bekannte Methoden hergestellt werden, z.B. durch die Umsetzung einer Verbindung der Formel III

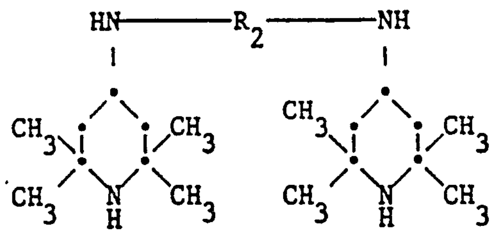

(III)

worin $R_2$ die oben angegebene Bedeutung hat, mit einem Chlorcarbonat der Formel IV

Cl-$COOR_1$ (IV)

worin $R_1$ die oben angegebene Bedeutung hat, in einem organischen Lösungsmittel in Anwesenheit einer organischen oder anorganischen Base, wie z.B. Pyridin, Triethylamin, Tributylamin, Natriumhydroxyd, Kaliumhydroxyd, Natriumcarbonat oder Kaliumcarbonat bei Temperaturen zwischen -30 und 30°C, bevorzugt zwischen -20 und 20°C.

Die Ausgangsprodukte der Formeln III und IV sind allgemein bekannt.

Zur besseren Veranschaulichung der vorliegenden Erfindung sind unten einige Herstellungsbeispiele für Verbindungen der Formel I angegeben; diese Beispiele dienen ausschliesslich der Erläuterung und bedeuten keinerlei Einschränkung der Erfindung.

**Beispiel 1:**

Herstellung von N,N'-Bis-(ethoxycarbonyl)-N,N'-bis-. (2,2,6,6-tetramethyl-piperidin-4-yl)-hexamethylendiamin.

22,8 g (0,21 Mol) Ethylchlorocarbonat werden einer auf -10°C abgekühlten Lösung von 39,4 g (0,1 Mol) N,N'-Bis-(2,2,6,6-tetramethyl-piperidin-4-yl)-hexamethylendiamin in 200 ml 1,2-Dichlorethan zugegeben. Die Temperatur soll dabei 0°C nicht übersteigen. Danach werden 8,4 g in 50 ml Wasser gelöstes Natriumhydroxyd langsam zugegeben, wobei die Temperatur bei 0°C gehalten wird. Dann wird die Temperatur auf 20°C steigen gelassen, die wässrige Phase abgetrennt und die organische Phase mit Wasser gewaschen. Nach der Trocknung über wasserfreiem $Na_2SO_4$ und Abdampfen des Lösungsmittels wird der Rückstand aus Octan

umkristallisiert. Das erhaltene Produkt weist einen Schmelzpunkt von 125°C auf.

Analyse für $C_{30}H_{58}N_4O_4$:.

Berechnet: C 66,87 % H 10,85 %. N 10,40 %

Gefunden : C 66,02 % ; H 10,73 % ; N 10,35 %

**Beispiele 2-12**:

Durch Wiederholen des Verfahrens gemäss Beispiel 1 mit entsprechenden Ausgangsprodukten werden folgende Verbindungen der Formel I erhalten:

| Beispiel Nr. | $R_1$ | $R_2$ | Smp. (°C) |
|---|---|---|---|
| 2 | Methyl | $-(CH_2)_2-$ | 200 |
| 3 | Ethyl | $-(CH_2)_2-$ | 161 |
| 4 | Isopropyl | $-(CH_2)_2-$ | 156 |
| 5 | n-Butyl | $-(CH_2)_2-$ | 124 |
| 6 | Allyl | $-(CH_2)_2-$ | 141 |
| 7 | Methyl | $-(CH_2)_3-$ | 130 |
| 8 | Ethyl | $-(CH_2)_3-$ | 84 |
| 9 | Methyl | $-(CH_2)_6-$ | 125 |
| 10 | Isopropyl | $-(CH_2)_6-$ | 116 |
| 11 | Ethyl | $-(CH_2)_2-N(COOC_2H_5)-(CH_2)_2-$ | 103 |
| 12 | Allyl | $-(CH_2)_6-$ | 91 |

Wie anfangs erwähnt, sind die Verbindungen der Formel I sehr wirksam bei der Verbesserung der Licht-, Wärme- und Oxydationsbeständigkeit von synthetischen Polymeren wie beispielsweise Polyäthylen hoher oder niedriger Dichte, Polypropylen, Aethylen/Propylencopolymeren, Aethylen/Vinylacetatcopolymeren, Polybutadien, Polyisopren, Polystyrol, Butadien/Styrolcopolymeren, Acrylnitril/Butadien/Styrolcopolymeren, Polymeren und Copolymeren von Vinylchlorid und Vinylidenchlorid, Polyoxymethylen, Polyurethanen, gesättigten und ungesättigten Polyestern, Polyamiden, Polycarbonaten, Polyacrylaten, Alkydharzen und Epoxidharzen.

Die Verbindungen der Formel I können im Gemisch mit synthetischen Polymeren in verschiedenen Anteilen eingesetzt werden, die von der Art des Polymeren, dem Endzweck und der Gegenwart weiterer Zusatzstoffe abhängen. Im allgemeinen setzt man zweckmässig 0,01 bis 5 Gew.-% der Verbindungen der Formel I, bezogen auf das Gewicht der Polymeren, vorzugsweise 0,1 bis 1%, ein.

Die Verbindungen der Formel I können nach verschiedenen Verfahren in die polymeren Materialien eingearbeitet werden, wie durch trockenes Einmischen in Pulverform oder nasses Einmischen in Form einer Lösung oder Suspension oder auch in Form eines konzentrierten Vorgemisches; das synthetische Polymer lässt sich dabei in Form von Pulver, Granulat, Lösung, Suspension oder Emulsion einsetzen. Die mit den Produkten der Formel I stabilisierten Polymeren lassen sich zur Herstellung von Formkörpern, Folien, Bändern, Endlosfäden, Lacken usw. verwenden.

Gegebenenfalls kann man den Mischungen von Verbindungen der Formel I mit den synthetischen Polymeren weitere Zusatzstoffe beigeben, wie beispielsweise Antioxydantien, UV-absorbierende Stoffe, Nickelstabilisatoren, Pigmente, Füllstoffe, Weichmacher, Antistatika, Flammschutzmittel, Schmierstoffe, Korrosionshemmstoffe sowie Metalldesaktivatoren. Beispiele für in Mischung mit Verbindungen der Formel I verwendbare Zusatzstoffe sind insbesondere:

## 1. Antioxidantien

### 1.1. Alkylierte Monophenole

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

### 1.2. Alkylierte Hydrochinone

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

### 1.3. Hydroxylierte Thiodiphenylether

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

### 1.4. Alkyliden-Bisphenole

2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methlyen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercapto-butan
Ethylenglycol-bis-(3,3-bis-(3'-tert.butyl-4'-hydroxyphenyl)-butyrat)
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

### 1.5 Benzylverbindungen

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat

1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester,
Calcium-salz.

**1.6. Acylaminophenole**

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

**1.7. Ester der β-(3,5-Di-tert.butyl-4-hydroxyphenyl)-propionsäure**

mit ein- oder mehrwertigen Alkoholen, wie z.B. mit
Methanol Diethylenglycol
Octadecanol Triethylenglycol
1,6-Hexandiol Pentaerythrit
Neopentylglycol Tris-hydroxyethyl-isocyanurat
Thiodiethylenglycol Di-hydroxyethyl-oxalsäurediamid

**1.8. Ester der β-(5-tert.butyl-4-hydroxy-3-methylphenyl)-propionsäure**

mit ein- oder mehrwertigen Alkoholen, wie z.B. mit
Methanol Diethylenglycol
Octadecanol Triethylenglycol
1,6-Hexandiol Pentaerythrit
Neopentylglycol Tris-hydroxyethyl-isocyanurat
Thiodiethylenglycol Di-hydroxyethyl-oxalsäurediamid

**1.9. Amide der β-(3,5-Di-tert.butyl-4-hydroxyphenyl)-propionsäure**

wie z.B.
N,N'-Di-(3,5-di-tert.butyl-4-hydroxyphenylpropionyl)-hexamethylen-diamin
N,N'-Di-(3,5-di-tert.butyl-4-hydroxyphenylpropionyl)-trimethylen-diamin
N,N'-Di-(3,5-di-tert.butyl-4-hydroxyphenylpropionyl)-hydrazin

**2. UV-Absorber und Lichtschutzmittel**

**2.1. 2-(2'-Hydroxyphenyl)-benztriazole,**

wie z.B. das 5'-Methyl-, 3',5'-Di-tert.butyl-, 5'-Tert.butyl-, 5'-(1,1,3,3-Tetramethyl-butyl)-, 5°Chlor-3',5'-di-tert.butyl-, 5°Chlor-3'-tert.butyl-5'-methyl-, 3'-sec.Butyl-5'-tert.butyl, 4'-Octoxy-, 3',5'-Di-tert.amyl- 3' 5'-Bis-(α,α-dimethylbenzyl)-Derivat.

**2.2. 2-Hydroxybenzophenone**

wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

**2.3. Ester von gegebenenfalls substituierten Benzoesäuren,**

wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexa-decylester.

**2.4.Acrylate,**

wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin,

**2.5. Nickelverbindungen,**

wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyldiethanolamin, Nickeldibutyldithiocarbamat, Nickelsalze von 4-Hydroxy-3,5-di-tert.butylbenzyl-phosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyra-zols, gegebenenfalls mit zusätzlichen Liganden.

**2.6. Sterisch gehinderte Amine,**

wie z.B. Bis-(2,2,6,6-tetramethyl-piperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester,
Kondensationsprodukt aus 1-Hydroxy-ethyl-2 2,6,6-Tetramethyl-4-hydroxypiperidin und Bernsteinsäure,
Kondensationsprodukt aus N,N'-(2,2,6,6-Tetramethyl-4-piperidyl)-hexamethylendiamin und 4-tert.Octylamino-2,6-dichlor-1,3,5-s-tri-azin,
Tris-(2,2,6,6-tetramethyl-4-piperidyl)-nitrilotriacetat, Tetrakis-(2,2,6,6-tetramethyl-4-piperidyl)-1,2,3,4-butantetracarbonsäure,
1,1 -(1,2-Ethandiyl)-bis-(3,3,5,5-tetramethyl-piperazinon).

**2.7. Oxalsäurediamide,**

wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

**3. Metalldesaktivatoren,**

wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzylidenoxalsäuredihydrazid.

**4. Phosphite und Phosphonite,**

wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdi-phosphit, Tristearylsorbit-triphosphit, Tetrakis-(2,4-di-tert.bu-tylphenyl)-4,4'-biphenylen-diphosphonit.

**5. Peroxidzerstörende Verbindungen,**

wie z.B. Ester der β-Thio-dipropionsäure, beispielsweise der Lauryl-, Stearyl-, Myristyl- oder Tridecylester, Mercaptobenzimidazol, das Zinksalz des 2-Mercapto-benzimidazols, Zink-dibutyl-dithiocarbamat, Dioctadecyldisulfid, Pentaerythrit-tetrakis-(β-dodecylmercapto)-propionat.

**6. Polyamidstabilisatoren,**

wie z.B. Kupfersalze in Kombination mit Jodiden und/oder Phosphorverbindungen und Salze des zweiwertigen Mangans.

**7. Basische Co-Stabilisatoren,**

wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

**8. Nukleierungsmittel,**

wie z.B. 4-tert.Butylbenzoesäure, Adipinsäure, Diphenylessigsäure.

**9. Füllstoffe und Verstärkungsmittel,**

wie z.B. Calciumcarbonat, Silikate, Glasfasern, Asbest, Talk, Kaolin, Glimmer, Bariumsulfat, Metalloxide und -hydroxide, Russ, Graphit.

**10. Sonstige Zusätze,**

wie z.B. Weichmacher, Gleitmittel, Emulgatoren, Pigmente, Optische Aufheller, Flammschutzmittel, Antistatika, Treibmittel.

Die Wirksamkeit der erfindungsgemäss einzusetzenden Produkte wird in den nachfolgenden Beispielen veranschaulicht, wo einige der in den Herstellungsbeispielen erhaltenen Produkte in einer synthetischen Polymerenmasse eingesetzt werden.

**Beispiel 13**

Je 0,5 g der in Tabelle 1 aufgeführten Stabilisatoren, 1 g Pentaerythrit-tetrakis-3-(3,5-di-t-butyl-4-hydroxyphenyl)-propionat, 1 g Calciumstearat, 1 g einer Pigmentpräparation ( ® Filofin Blue 600 der CIBA-GEIGY AG) werden mit 1000 g Polypropylen mit einem Schmelzindex von 3,0 ( ® Propathen HF 18, der Firma Imperial Chemical Industries) innig vermischt. Das erhaltene Gemisch wird dann bei einer Temperatur zwischen 200 und 220 °C extrudiert und zu Granulat zerschnitten, aus dem man durch Formpressen bei 250 °C Platten von 2 mm Dicke erhält. Die Platten werden in einem Weather-Ometer 65 WR (ASTM G 27-70) mit einer Temperatur der schwarzen Tafel von 63°C ausgesetzt, bis zum eben wahrnehmbaren Beschlagens der Oberfläche (chalking). Die dazu erforderliche Zeit (in Stunden) ist in Tabelle 1 als Mass für die Wirksamkeit als Stabilisator angegeben.

**Tabelle 1**

| Stabilisator | Zeit bis zum Beschlagen (in Stunden) |
|---|---|
| keiner | 500 |
| Verbindung von Bsp. 1 | 1810 |
| Verbindung von Bsp. 4 | 1600 |

**Beispiel 14**

Polypropylenplatten werden genau wie in Beispiel 13 beschrieben hergestellt und getestet, mit der einzigen Ausnahme, dass statt 0,5 g nun 1,0 g des erfindungsgemäss einzusetzenden Stabilisators eingesetzt wird. Die Resultate sind in Tabelle 2 wiedergegeben.

**Tabelle 2**

| Stabilisator | Zeit bis zum Beschlagen (in Stunden) |
|---|---|
| keiner | 500 |
| Verbindung von Bsp. 1 | 2,850 |
| Verbindung von Bsp. 2 | 2,690 |
| Verbindung von Bsp. 3 | 2,850 |
| Verbindung von Bsp. 5 | 2,505 |
| Verbindung von Bsp. 7 | 2,940 |
| Verbindung von Bsp. 8 | 2,690 |
| Verbindung von Bsp. 9 | 2,850 |
| Verbindung von Bsp. 10 | 2,690 |
| Verbindung von Bsp. 11 | 2,510 |

**Patentansprüche**

1. Verwendung einer Verbindung der Formel I

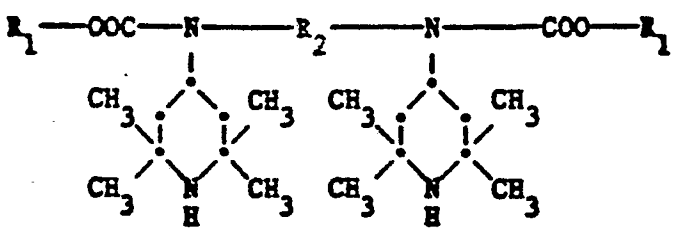

(I)

worin $R_1$ $C_1$-$C_{12}$ Alkyl, $C_3$-$C_{12}$ Alkenyl, $C_5$-$C_{12}$ Cycloalkyl, unsubstituiertes oder durch 1 bis 3 $C_1$-$C_4$ Alkylgruppen substituiertes Phenyl oder Hydroxyphenyl, unsubstituiertes oder durch 1 bis 3 $C_1$-$C_4$ Alkylgruppen substituiertes Benzyl oder Hydroxybenzyl ist und $R_2$ unsubstituiertes oder durch -OH substituiertes $C_1$-$C_{18}$ Alkylen, $C_5$-$C_{18}$ Cycloalkylen, $C_6$-$C_{18}$ Arylen, $C_7$-$C_{18}$ Aralkylen oder eine Gruppe der Formel II

$$-R_3-(X-R_4)_n- \quad (II)$$

bedeutet, worin $R_3$ und $R_4$ unabhängig voneinander $C_2$-$C_6$ Alkylen sind, n 1 oder 2 bedeutet und X -O- oder -N($R_5$)- ist, wobei $R_5$ $C_1$-$C_{12}$ Alkyl, $C_5$-$C_{12}$ Cycloalkyl, $C_6$-$C_{12}$ Aryl, $C_7$-$C_{12}$ Aralkyl, 2,2,6,6-Tetramethyl-piperidin-4-yl oder eine Gruppe -COOR$_1$, worin $R_1$ die oben angegebene Bedeutung hat, ist, zum Stabilisieren von synthetischen Polymeren gegen die Einwirkung von Licht, Wärme und Sauerstoff.

2. Verwendung gemäss Anspruch 1, dadurch gekennzeichnet, dass eine Verbindung der Formel I eingesetzt wird, worin $R_1$ $C_1$-$C_8$ Alkyl, $C_3$-$C_8$ Alkenyl, Cyclohexyl, Phenyl oder Benzyl ist, $R_2$ $C_1$-$C_6$ Alkylen, Cycloalkylen, Phenylen, Xylylen oder eine Gruppe der Formel II ist, worin $R_3$ und $R_4$ $C_2$-C Alkylen sind und $R_5$ eine Gruppe -COOR$_1$ bedeutet, worin $R_1$ die in diesem Anspruch bereits angegebene Bedeutung hat.

3. Verwendung gemäss Anspruch 1, dadurch gekennzeichnet, dass eine Verbindung der Formel I eingesetzt wird, worin $R_1$ $C_1$-$C_4$ Alkyl und $R_2$ $C_2$-$C_6$ Alkylen bedeuten.

4. Verwendung gemäss Anspruch 1, dadurch gekennzeichnet, dass die Verbindung der Formel I N,N'-Bis-(methoxycarbonyl)-N,N'-bis-(2,2,6,6-tetramethyl-piperidin-4-yl)-hexamethylendiamin ist.

5. Verwendung gemäss Anspruch 1, dadurch gekennzeichnet, dass die Verbindung der Formel I N,N'-Bis-(ethoxycarbonyl)-N,N'-bis(2,2,6,6-tetramethyl-piperidin-4-yl)-hexamethylendiamin ist.

6. Stoffzusammensetzung enthaltend ein synthetisches Polymer und einen oder mehrere Stabilisatoren der Formel I in einer Menge zwischen 0,01 und 5 Gew.-% bezogen auf das synthetische Polymere.

7. Stoffzusammensetzung gemäss Anspruch 6, dadurch gekennzeichnet, dass sie zusätzlich Antioxydantien, UV-absorbierende Stoffe, Nickelstabilisatoren, Pigmente, Füllstoffe, Weichmacher, Antistatika, Flammschutzmittel, Schmierstoffe, Korrosionshemmstoffe und/oder Metalldesaktivatoren enthält.

8. Stoffzusammensetzung gemäss Anspruch 6, dadurch gekennzeichnet, dass als synthetisches Polymer Polyethylen oder Polypropylen vorliegt.

9. Verbindung der Formel I

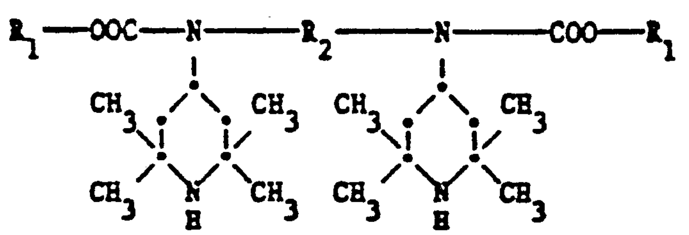

(I)

worin $R_1$ $C_3$-$C_{12}$ Alkyl, $C_3$-$C_{12}$ Alkenyl, $C_5$-$C_{12}$ Cycloalkyl, unsubstituiertes oder durch 1 bis 3 $C_1$-$C_4$ Alkylgruppen substituiertes Phenyl oder Hydroxyphenyl oder unsubstituiertes oder durch 1 bis 3 $C_1$-$C_4$ Alkylgruppen substituiertes Benzyl oder Hydroxybenzyl ist und $R_2$ unsubstituiertes oder durch -OH substituiertes $C_1$-$C_{18}$ Alkylen, $C_5$-$C_{18}$ Cycloalkylen, $C_6$-$C_{18}$ Arylen, $C_7$-$C_{18}$ Aralkylen oder eine Gruppe der Formel II

$$-R_3-(X-R_4)_n- \qquad (II)$$

bedeutet, worin $R_3$ und $R_4$ unabhängig voneinander $C_2$-$C_6$ Alkylen sind, n 1 oder 2 bedeutet und X -O- oder -N($R_5$)- ist, wobei $R_5$ $C_1$-$C_{12}$ Alkyl, $C_5$-$C_{12}$ Cycloalkyl, $C_6$-$C_{12}$ Aryl, $C_7$-$C_{12}$ Aralkyl, 2,2,6,6-Tetramethyl-piperidin-4-yl oder eine Gruppe -COO$R_1$ bedeutet, worin $R_1$ die oben angegebene Bedeutung hat.

## Claims

1. Use of a compound of the formula (I)

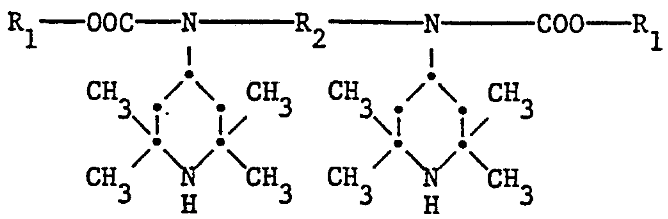

(I)

in which $R_1$ is $C_1$-$C_{12}$alkyl, $C_3$-$C_{12}$alkenyl, $C_5$-$C_{12}$cycloalkyl, or phenyl or hydroxyphenyl, each unsubstituted or substituted by 1 to 3 $C_1$-$C_4$-alkyl groups, or benzyl or hydroxybenzyl, each unsubstituted or substituted by 1 to 3 $C_1$-$C_4$alkyl groups, $R_2$ is unsubstituted or OH-substituted $C_1$-$C_{18}$alkylene, $C_5$-$C_{18}$cycloalkylene, $C_6$-$C_{18}$arylene, $C_7$-$C_{18}$-aralkylene or a radical of the formula (II)

$$-R_3-(X-R_4)_n \qquad (II)$$

in which $R_3$ and $R_4$ are each independently of the other $C_2$-$C_6$alkylene, n is 1 or 2 and X is -O- or -$N_R$-, with $R_5$ being $C_1$-$C_{12}$alkyl, $C_5$-$C_{12}$-cycloalkyl, $C_6$-$C_{12}$aryl, $C_7$-$C_{12}$aralkyl, 2,2,6,6-tetramethylpiperidin-4-yl or a radical -COO$R_1$ in which $R_1$ is as defined above, for stabilising synthetic polymers against the action of light, heat and oxygen.

2. Use according to claim 1 of a compound of formula (I) wherein $R_1$ is $C_1$-$C_8$alkyl, $C_3$-$C_8$alkenyl, cyclohexyl, phenyl or benzyl, $R_2$ is $C_1$-$C_6$alkylene, cycloalkylene, phenylene, xylylene or a group of formula II, wherein $R_3$ and $R_4$ are $C_2$-$C_4$alkylene and $R_5$ is a radical -COO$R_1$ in which $R_1$ is as defined above.

3. Use according to claim 1 of a compound of formula (I), wherein $R_1$ is $C_1$-$C_4$alkyl and $R_2$ is $C_2$-$C_6$alkylene.

4. Use according to claim 1, wherein the compound of formula (I) is N,N'-bis(methoxycarbonyl)-N,N'-bis(2,2,6,6-tetramethylpiperidin-4-yl)-hexamethylenediamine.

5. Use according to claim 1, wherein the compound of formula (I) is N,N'-bis(ethoxycarbonyl)-N,N'-bis(2,2,6,6-tetramethylpiperidin-4-yl)-hexamethylenediamine.

6. A composition which comprises a synthetic polymer and one or more of the stabilisers of the formula (I) in an amount of 0.01 to 5% by weight, based on the weight of the synthetic polymer.

7. A composition according to claim 6, which additionally contains antioxidants, UV absorbents, nickel stabilisers, pigments, fillers, plasticisers, anti-static agents, flame retardants, lubricants, corrosion inhibitors and/or metal passivators.

8. A composition according to claim 6, wherein the synthetic polymer is polyethylene or polypropylene.

9. A compound of the formula (I)

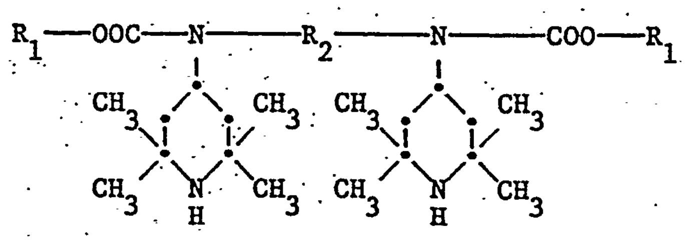

(I)

in which $R_1$ is $C_3$-$C_{12}$alkyl, $C_3$-$C_{12}$alkenyl, $C_5$ -$C_{12}$cycloalkyl, or phenyl or hydroxyphenyl, each unsubstituted or substituted by 1 to 3 $C_1$-$C_4$-alkyl groups, or benzyl or hydroxybenzyl, each unsubstituted or substituted by 1 to 3 $C_1$-$C_4$alkyl groups, $R_2$ is unsubstituted or OH-substituted $C_1$-$C_{18}$alkylene, $C_5$-$C_{18}$cycloalkylene, $C_6$-$C_{18}$arylene, $C_7$-$C_{18}$-aralkylene or a radical of the formula (II)

$$-R_3-(X-R_4)_n- \quad (II)$$

in which $R_3$ and $R_4$ each independently of the other are $C_2$-$C_6$alkylene, n is 1 or 2 and X is -O- or

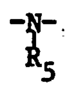

with $R_5$ being $C_1$-$C_{12}$alkyl, $C_5$-$C_{12}$-cycloalkyl, $C_6$-$C_{12}$aryl, $C_7$-$C_{12}$aralkyl, 2,2,6,6-tetramethylpiperidin-4-yl or a radical -COOR$_1$ in which $R_1$ is as defined above.

**Revendications**

1. Application d'un composé répondant à la formule I:

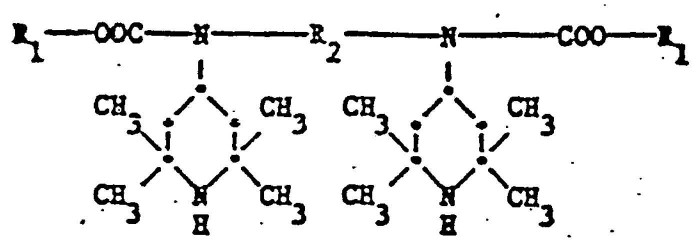

(I)

dans laquelle

$R_1$ représente un alkyle en $C_1$-$C_{12}$, un alcényle en $C_3$-$C_{12}$, un cycloalkyle en $C_5$-$C_{12}$, un radical phényle ou hydroxyphényle non substitué ou porteur d'un à trois alkyles en $C_1$-$C_4$, ou un radical benzyle ou hydroxybenzyle non substitué ou porteur d'un à trois alkyles en $C_1$-$C_4$, et

$R_2$ représente un alkylène en $C_1$-$C_{18}$ non substitué ou porteur d'un -OH, un cycloalkylène en $C_5$-$C_{18}$, un arylène en $C_6$-$C_{18}$, un aralkylène en $C_7$-$C_{18}$ ou un radical répondant à la formule II :

$$-R_3-(X-R_4)_n- \qquad (II)$$

dans laquelle

$R_3$ et $R_4$ représentent chacun, indépendamment l'un de l'autre, un alkylène en $C_2$-$C_6$, n est égal à 1 ou à 2 et X représente -O- ou un radical -N($R_5$)- dont le symbole $R_5$ désigne un alkyle en $C_1$-$C_{12}$, un cycloalkyle en $C_5$-$C_{12}$, un aryle en $C_6$-$C_{12}$, un aralkyle en $C_7$-$C_{12}$, un tétraméthyl-2,2,6,6 pipéridyle-4 ou un radical -COOR$_1$ dont le symbole $R_1$ a la signification précédemment donnée,

à la stabilisation de polymères synthétiques contre l'action de la lumière, de la chaleur et de l'oxygène.

2. Application selon la revendication 1 caractérisée en ce qu'on utilise un composé de formule I dans lequel $R_1$ représente un alkyle en $C_1$-$C_8$, un alcényle en $C_3$-$C_8$, un cyclohexyle, un phényle ou un benzyle et $R_2$ un alkylène en $C_1$-$C_6$, un cycloalkylène, un phénylène, un xylylène, ou un radical de formule II dans lequel $R_3$ et $R_4$ représentent chacun un alkylène en $C_2$-$C_4$ et $R_5$ un radical -COOR$_1$ dont le symbole $R_1$ a la signification qui vient de lui être donnée dans cette même revendication.

3. Application selon la revendication 1 caractérisée en ce qu'on utilise un composé de formule 1 dans lequel $R_1$ représente un alkyle en $C_1$-$C_4$ et $R_2$ un alkylène en $C_2$-$C_6$.

4. Application selon la revendication 1 caractérisée en ce que le composé de formule I est la N,N'-bis-(méthoxycarbonyl)-N,N'-bis-(tétraméthyl-2,2,6,6 pipéridyl-4)-hexaméthylène-diamine.

5. Application selon la revendication 1 caractérisée en ce que le composé de formule I est la N,N'-bis-(éthoxycarbonyl)-N,N'-bis-(tétraméthyl-2,2,6,6 pipéridyl-4)-hexaméthylène-diamine.

6. Composition renfermant un polymère synthétique et un ou plusieurs stabilisants de formule I en une quantité comprise entre 0,01 et 5 % en poids par rapport au polymère synthétique.

7. Composition selon la revendication 6 caractérisée en ce qu'elle contient en outre des anti-oxydants, des absorbeurs de rayons ultra-violets, des stabilisants au nickel, des pigments, des charges, des plastifiants, des anti-statiques, des ignifugeants, des lubrifiants, des inhibiteurs de corrosion et/ou des désactivants de métaux.

8. Composition selon la revendication 6 caractérisée en ce qu'elle contient, comme polymère synthétique, du polyéthylène ou du polypropylène.

9. Composé répondant à la formule I :

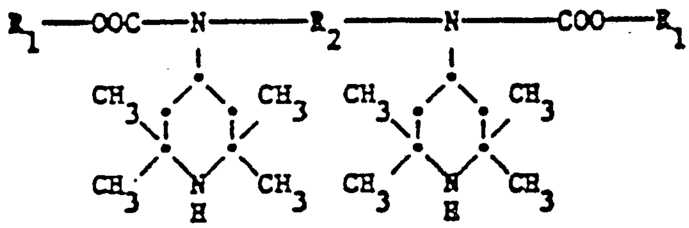

(I)

dans laquelle

$R_1$ représente un alkyle en $C_3$-$C_{12}$, un alcényle en $C_3$-$C_{12}$, un cycloalkyle en $C_5$-$C_{12}$, un radical phényle ou hydroxyphényle non substitué ou porteur d'un à trois alkyles en $C_1$-$C_4$, ou un radical benzyle ou hydroxybenzyle non substitué ou porteur d'un à trois alkyles en $C_1$-$C_4$, et

$R_2$ représente un alkylène en $C_1$-$C_{18}$ non substitué ou porteur d'un -OH, un cycloalkylène en $C_5$-$C_{18}$, un arylène en $C_6$-$C_{18}$, un aralkylène en $C_7$-$C_{18}$ ou un radical répondant à la formule II :

$$-R_3-(X-R_4-)_n- \qquad (II)$$

dans laquelle

$R_3$ et $R_4$ représentent chacun, indépendamment l'un de l'autre, un alkylène en $C_2$-$C_6$, n est égal à 1 ou à 2 et X représente -O- ou un radical -N($R_5$)- dont le symbole $R_5$ désigne un alkyle en $C_1$-$C_{12}$, un cycloalkyle en $C_5$-$C_{12}$, un aryle en $C_6$-$C_{12}$, un aralkyle en $C_7$-$C_{12}$, un tétraméthyl-2,2,6,6 pipéridyle-4 ou un radical -COOR$_1$ dont le symbole $R_1$ a la signification précédemment donnée.